Europäisches Patentamt

European Patent Office

Office européen des brevets

(19)

(11) Veröffentlichungsnummer: **0 185 202 B1**

(12) **EUROPÄISCHE PATENTSCHRIFT**

(45) Veröffentlichungstag der Patentschrift:
**31.01.90**

(21) Anmeldenummer: **85114519.3**

(22) Anmeldetag: **15.11.85**

(51) Int. Cl.⁴: **C 07 C 7/04, F 25 J 3/08**

(54) Verfahren zur Gewinnung von C2+-, C3+- oder von C4+- Kohlenwasserstoffen.

(30) Priorität: **17.12.84 DE 3445995**

(43) Veröffentlichungstag der Anmeldung:
**25.06.86 Patentblatt 86/26**

(45) Bekanntmachung des Hinweises auf die Patenterteilung:
**31.01.90 Patentblatt 90/5**

(84) Benannte Vertragsstaaten:
**DE FR GB IT NL SE**

(56) Entgegenhaltungen:
**DE-A- 3 408 760**
**GB-A- 2 102 931**
**US-A- 4 336 045**

(73) Patentinhaber: **Linde Aktiengesellschaft,**
**Abraham-Lincoln-Strasse 21, D-6200 Wiesbaden (DE)**

(72) Erfinder: **Bauer, Heinz, Dr. rer. nat., Nibelungenstrasse 6,**
**D-8000 München 19 (DE)**

(74) Vertreter: **Schaefer, Gerhard, Dr., Linde**
**Aktiengesellschaft Zentrale Patentabteilung,**
**D-8023 Höllriegelskreuth (DE)**

**Beschreibung**

Die Erfindung betrifft ein Verfahren zur Abtrennung von $C_{2+}$-, $C_{3+}$- oder von $C_{4+}$-Kohlenwasserstoffen aus einem leichte Kohlenwasserstoffe und gegebenenfalls leichter als Methan siedende Komponenten enthaltenden Gasstrom, bei dem der unter erhöhtem Druck stehende Gasstrom abgekühlt, partiell kondensiert und in einem Abscheider in eine flüssige und eine gasförmige Fraktion getrennt wird, bei dem die gasförmige Fraktion arbeitsleistend entspannt und die flüssige Fraktion durch Rektifikation in einem im wesentlichen $C_{2+}$-, $C_{3+}$- bzw. $C_{4+}$-Kohlenwasserstoff enthaltenden Produktstrom und einen überwiegend leichter siedende Komponenten enthaltenden Restgasstrom zerlegt wird und bei dem die nach der partiellen Kondensation anfallende gasförmige Fraktion vor der arbeitsleistenden Entspannung durch Wärmetausch mit der arbeitsleistend entspannten gasförmigen Fraktion weiter abgekühlt wird und die dabei zusätzlich auskondensierenden Komponenten vor der arbeitsleistenden Entspannung abgetrennt werden.

Derartige Verfahren dienen vor allem zur Abtrennung von Ethan oder von Propan aus Erdgasen oder anderen Gasen, beispielsweise Raffinerieabgasen. Ausserdem eignen sich diese Verfahren zur Abtrennung von vergleichbaren ungesättigten Kohlenwasserstoffen, also beispielsweise von Ethylen oder Propylen, sofern diese Komponenten im zu zerlegenden Gasgemisch enthalten sind, was beispielsweise bei Raffinerieabgasen der Fall sein kann. Die Weiterverarbeitung von Raffinerieabgasen ist in letzter Zeit wirtschaftlich interessant geworden, da die Marktpreise für LPG ($C_3/C_4$-Kohlenwasserstoffgemisch) gestiegen sind, während umgekehrt Vakuumrückstände sowie Schweröl schlecht verkäuflich sind. Aus diesem Grund werden schlecht vermarktbare schwere Produkte zur Deckung des internen Brennstoffbedarfs einer Raffinerie verfeuert, während gut verkäufliche $C_{3+}$-Kohlenwasserstoffe aus dem Abgas, das insbesondere bei der Verarbeitung von leichten Rohölbestandteilen zu Benzin in grossen Mengen anfällt, abgetrennt werden.

In der älteren, nicht vorveröffentlichten deutschen Patentanmeldung P 3 408 760.5 ist bereits ein Verfahren der eingangs genannten Art beschrieben, das sich auf die Abtrennung von $C_{3+}$-Kohlenwasserstoffen bezieht. Das wesentliche Merkmal dieser älteren Anmeldung besteht darin, dass die bei der arbeitsleistenden Entspannung der nach der partiellen Kondensation verbliebenen gasförmigen Fraktion gewonnene Kälte nicht zur Erzeugung von Rücklaufflüssigkeit bei der Rektifikation eingesetzt wird, sondern zur Abkühlung und partiellen Kondensation des Rohgases. Es ist daher nicht mehr erforderlich, die leichten Anteile des Gasstroms in die Rektifikation zu führen. Der Verzicht auf die Einleitung der leichten Anteile des Einsatzstroms (insbesondere Wasserstoff sowie $C_1$- und gegebenenfalls $C_2$-Kohlenwasserstoffe bei Raffineriegasen, insbesondere Stickstoff sowie $C_1$- und gegebenenfalls $C_2$-Kohlenwasserstoffe bei Erdgasen) in die Trennsäule ermöglicht es, die Rektifikation auf einem höheren Temperaturniveau durchzuführen. Die Möglichkeit, einfache und preiswerte Fremdkältekreisläufe zur Kopfkühlung bei der Rektifikation zu verwenden, bedeutet zwar eine erhebliche Verbesserung der Verfahrensführung, eine weitere Verbesserung ist aber dennoch wünschenswert.

In der GB-A 2 102 931 ist ein weiteres Verfahren der eingangs genannten Art beschrieben, bei dem kondensierbare Kohlenwasserstoffe aus einem Gas abgetrennt werden. Unter erhöhtem Druck stehendes, partiell kondensiertes Rohgas wird einer Phasentrennung in einem ersten Abscheider unterworfen. Die dabei gewonnene flüssige Fraktion wird einer Rektifikation zugeführt, während die gasförmige Fraktion durch indirekten Wärmeaustausch weiter abgekühlt und arbeitsleistend entspannt wird. Der entspannte Gasstrom wird zur Abkühlung der nicht entspannten gasförmigen Fraktion eingesetzt. Die bei der Abkühlung aus der gasförmigen Fraktion zusätzlich auskondensierten Bestandteile werden vor der Entspannung in einem weiteren Abscheider abgetrennt.

Durch den Wärmetausch zwischen entspannter und nicht entspannter gasförmiger Fraktion wird die bei der arbeitsleistenden Entspannung gewonnene Kälte vorteilhaft zur Bildung von Kondensat ausgenutzt. Allerdings muss dieses Kondensat in einem zusätzlichen Abscheider abgetrennt werden, was einen für diesen Zweck relativ hohen apparativen Aufwand erfordert. Die zweimalige Phasentrennung ist ausserdem ein verglichen zur Komplexität der Apparatur wenig effektives Trennverfahren.

Der Erfindung liegt deshalb die Aufgabe zugrunde, ein Verfahren der eingangs genannten Art so auszugestalten, dass die Abtrennung von $C_{2+}$-, $C_{3+}$- bzw. $C_{4+}$-Kohlenwasserstoffen mit verringertem Aufwand ermöglicht wird.

Diese Aufgabe wird dadurch gelöst, dass der Abscheider, in dem die Trennung in die flüssige und die gasförmige Fraktion durchgeführt wird, mindestens zwei Gleichgewichtsstufen aufweist und dass die zusätzlich auskondensierten Komponenten im Gegenstrom zu der in dem Abscheider aufsteigenden gasförmigen Fraktion in dem unteren Bereich des Abscheiders fallen.

Die durch die Abkühlung im Wärmetausch mit der entspannten gasförmigen Fraktion kondensierte Flüssigkeit wird innerhalb des Abscheiders, der ohnehin zur Abtrennung der gasförmigen Fraktion aus dem partiell kondensierten Gasstrom benötigt wird, im Gegenstrom zu der aufsteigenden gasförmigen Fraktion geführt. Die Spitzenkälte aus der Entspannung wird also nicht nur zur Kondensatbildung eingesetzt, sondern durch Stoffaustausch innerhalb des Abscheiders wird zusätzlich eine rektifikatorische Wirkung erreicht und damit die Effektivität der Phasentrennung deutlich verbessert. Weitere in der aufsteigenden gasförmigen Fraktion enthaltene kondensierbare Bestandteile werden durch nach unten strömende Kondensat ausgewaschen und mit der flüssigen Fraktion abgezogen, stehen so für die Rektifikation zur Verfügung. Gleichzeitig verdampfen unerwünschte leichte Komponenten, die in dem zusätzlich gebildeten Kondensat enthalten sind. '

In bevorzugter Ausgestaltung des erfindungsgemässen Verfahrens wird der Wärmetausch zwischen der nicht entspannten und der entspannten gasförmigen Fraktion im oberen Bereich des Abscheiders durchgeführt.

Die zwei Gleichgewichtsstufen innerhalb des Abscheiders werden durch den Wärmeentzug mit Hilfe der entspannten gasförmigen Fraktion, der einer Kopfkühlung in einer Trennsäule entspricht, erzeugt. Innerhalb des Abscheiders finden sowohl die Abtrennung der bei der partiellen Kondensation gebildeten flüssigen Fraktion, als auch die Abtrennung der zusätzlich auskondensierten Komponenten statt, wobei der partiell kondensierte Gasstrom in den unteren Bereich der Kolonne eingespeist und der Wärmetausch zwischen der nicht entspannten und der entspannten gasförmigen Fraktion im oberen Bereich der Kolonne erfolgt. Die Einspeisung des partiell kondensierten Gasstroms in den unteren Bereich der Kolonne bewirkt einen Wärmeeintrag in den Kolonnensumpf, wodurch in der flüssigen Fraktion gelöste leichte Bestandteile zumindest teilweise wieder abgestrippt und somit von der Rektifikation ferngehalten werden. Der im Kolonnenkopf erfolgende Wärmetausch zwischen der kalten entspannten und der noch nicht entspannten gasförmigen Fraktion bewirkt dagegen eine Kopfkühlung, die zu einem verstärkten Kondensieren höhersiedender Anteile der gasförmigen Fraktion und damit zu einer besseren Ausbeute führt.

Diese Ausgestaltung der Erfindung erfordert in der Regel im oberen Bereich des für die Phasentrennung nach der partiellen Kondensation vorgesehenen Abscheiders einen gewickelten Wärmetauscher oder andere äquivalente konstruktive Massnahmen, durch die ein Wärme- und Stoffaustausch in diesem Bereich bewirkt wird. Bei der Verarbeitung sehr grosser Gasmengen werden die dafür erforderlichen Investitionskosten so hoch, dass sich dann in vielen Fällen eine andere Ausführungsform der Erfindung als kostengünstiger erweist.

Gemäss dieser Ausführungsform ist vorgesehen, dass der Wärmetausch zwischen der nicht entspannten und der entspannten gasförmigen Fraktion ausserhalb des Abscheiders durchgeführt wird und die dabei zusätzlich auskondensierten Komponenten in den Abscheider zurückgeführt werden.

Obwohl diese Ausführungsform der Erfindung einen zusätzlichen Abscheider benötigt, ist sie bei grossen Anlagen kostengünstiger, da statt eines teuren gewickelten Wärmetauschers oder anderer ebenfalls aufwendiger Konstruktionen im oberen Bereich des Abscheiders die weitere Abkühlung der gasförmigen Fraktion in einem vergleichsweise billigen Plattenwärmetauscher erfolgen kann. Unabhängig von der Anlagengrösse hat diese Ausführungsform darüber hinaus noch den Vorteil, für die Abtrennung von $C_{4+}$-Kohlenwasserstoffen besser geeignet zu sein als die zuvor beschriebene Variante der Erfindung.

Da bei dieser Ausführungsform der Erfindung der Rücklauf für den Rektifikationsabschnitt innerhalb des Abscheiders separat gebildet wird und nicht direkt im Abscheider, ergeben sich bei dieser Ausführungsform auch Vorteile bezüglich der Rücklaufverhältnisse im Abscheider, insbesondere dadurch, dass bei der tiefsten Temperatur bereits die volle Rücklaufmenge aufgegeben wird.

Um thermische Irreversibilitäten im Abscheider zu vermeiden, ist es in weiterer Ausgestaltung dieser Ausführungsform der Erfindung vorteilhaft, dass die zusätzlich auskondensierten Komponenten vor der Rückführung in den Abscheider teilweise wieder erwärmt werden. Damit findet im Abscheider im wesentlichen nur noch ein Stoffaustausch, jedoch kaum noch ein Wärmeaustausch statt. Als zweckmässig hat sich dabei erwiesen, die zusätzlich auskondensierten Komponenten mindestens bis zum Siedepunkt zu erwärmen. Dies kann beispielsweise im Wärmetausch mit der weiter abzukühlenden gasförmigen Fraktion vor deren arbeitsleistender Entspannung erfolgen.

Bei der Verarbeitung von Gasströmen, die reich an leichter als Methan siedenden Komponenten sind, ist in Weiterbildung der Erfindung vorgesehen, dass eine Anreicherung dieser Komponenten erfolgen kann, wozu vor der arbeitsleistenden Entspannung der gasförmigen Fraktion aus dieser $C_1$- und $C_2$-Kohlenwasserstoffe durch partielle Kondensation abgetrennt werden. Diese Verfahrensweise kann beispielsweise bei der Abtrennung von $C_{2+}$- bzw. $C_{3+}$-Kohlenwasserstoffen und von Stickstoff aus stickstoffreichem Erdgas oder insbesondere zur Gewinnung der genannten schweren Kohlenwasserstoffe und von Wasserstoff aus wasserstoffreichen Raffinerieabgasen zum Einsatz kommen. Eine solche Abtrennung ist insbesondere dann von Vorteil, wenn der Einsatzstrom einen relativ hohen Anteil tiefsiedender Komponenten aufweist, beispielsweise einen Wasserstoffgehalt in der Grössenordnung von 50 bis 90%. Eine solche Wasserstoffmenge reicht nämlich aus, die für die zusätzliche Abtrennung benötigte Kälte bei der Entspannung zu erzeugen, ohne dass zusätzlich Fremdenergie aufgewendet werden muss.

In vielen Anwendungsfällen ist eine weitere Zerlegung des $C_{2+}$- bzw. $C_{3+}$-Kohlenwasserstoffprodukts, insbesondere eine Trennung zwischen einem $C_3/C_4$-Kohlenwasserstoffgemisch und $C_{5+}$-Kohlenwasserstoffen erwünscht. Zu diesem Zweck wird gemäss einer bevorzugten Ausgestaltung des erfindungsgemässen Verfahrens vor der Bildung der flüssigen und der gasförmigen Fraktion ein Grossteil der $C_{5+}$-Kohlenwasserstoffe aus dem Gasstrom abgetrennt, sofern die Konzentration dieser Bestandteile so gross ist, dass sich eine derartige Abtrennung lohnt.

Die $C_{5+}$-Abtrennung erfolgt zweckmässig durch partielle Kondensation bei einer Temperatur oberhalb der Temperatur, bei der die oben erwähnte flüssigen und gasförmigen Fraktionen gebildet werden. Durch die vorherige Abtrennung der schweren Komponenten ist das der Rektifikation zugeführte Gemisch nahezu frei an $C_{5+}$-Kohlenwasserstoffen, so dass bei der nachfolgenden Rektifikation der flüssigen Fraktion ein Produktstrom gewonnen wird, der bei einer $C_{3+}$-Abtrennung eine handelsübliche LPG-Fraktion bildet.

Um die Ausbeute von $C_3$- und $C_4$-Kohlenwasserstoffen zu erhöhen, wird in weiterer Ausbildung dieser Verfahrensvariante vorgeschlagen, dass die abgetrennten schweren Kohlenwasserstoffe ebenfalls der Rektifikation zugeführt werden, wobei die Einspeisung der $C_{5+}$-Fraktion in eine Rektifiziersäule entsprechend dem Gleichgewichtsverlauf in der Rektifiziersäule unterhalb der Einspeisung der bei der partiellen Kondensation gebildeten flüssigen Fraktion erfolgt und wobei ferner vorgesehen ist, dass ein im wesentlichen $C_3$- und $C_4$-Kohlenwasserstoffe enthaltender Strom zwischen den beiden Einspeisungen entnommen wird. Durch die zusätzliche Rektifikation der $C_{5+}$-Fraktion werden auch noch die $C_3/C_4$-Kohlenwasserstoffe, die bei der Bildung der $C_{5+}$-Fraktion durch partielle Kondensation in Lösung gegangen sind, als Produkt zurückgewonnen. Zwischen den beiden Einspeisestellen bildet innerhalb der Rektifiziersäule ein Bereich maximaler $C_3/C_4$-Konzentration aus, wo in günstiger Weise der $C_3/C_4$-Produktstrom entnommen wird.

Weitere Einzelheiten der Erfindung werden nachfolgend anhand der in den Figuren schematisch dargestellten Ausführungsbeispiele erläutert.

Es zeigen:

Fig. 1 eine Prinzipskizze des erfindungsgemässen Verfahrens,

Fig. 2 eine erste Ausführungsform der Erfindung, bei der der Druck des zu zerlegenden Gasgemisches höher als der Rektifikationsdruck ist,

Fig. 3 eine weitere Ausführungsform der Erfindung, bei der der Rektifikationsdruck höher als der Druck des zu zerlegenden Gasgemisches ist,

Fig. 4 eine Variante des erfindungsgemässen Verfahrens mit einer Vorabtrennung von $C_{5+}$-Kohlenwasserstoffen,

Fig. 5 eine weitere Ausführungsvariante, bei der aus der gasförmigen Fraktion auskondensierte Komponenten zurückgeführt werden und

Fig. 6 und 7 weitere Ausführungsformen der Erfindung mit einer Rückführung zusätzlich auskondensierter Komponenten.

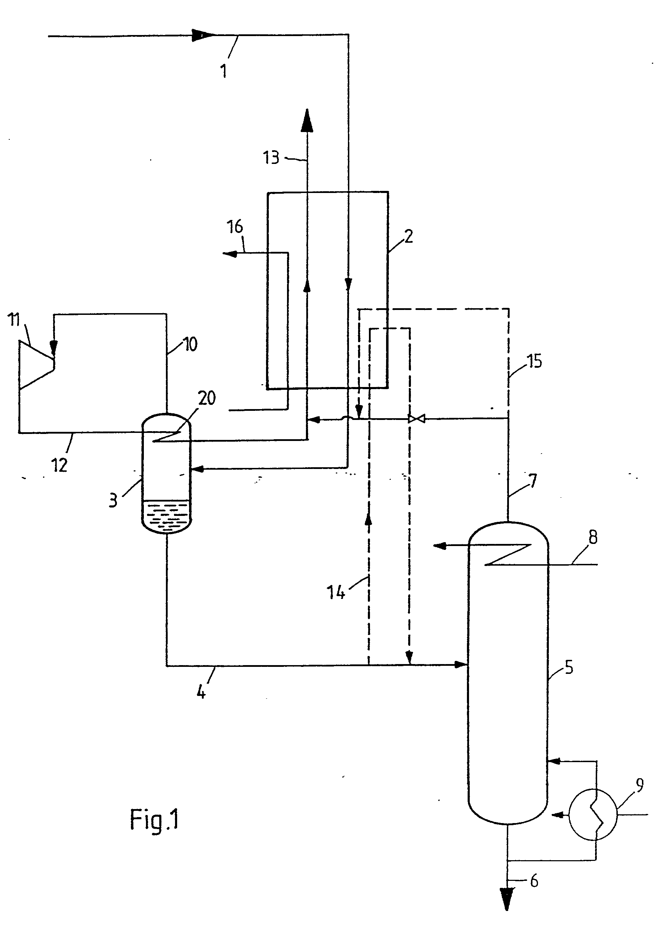

Beim in der Fig. 1 dargestellten Ausführungsbeispiel wird über Leitung 1 der zu zerlegende Gasstrom unter erhöhtem Druck und bei annähernd Umgebungstemperatur einem Wärmetauscher 2 zugeführt, in dem er so weit abgekühlt wird, dass der grösste Teil der abzutrennenden Kohlenwasserstoffe, also der $C_{2+}$-, $C_{3+}$- bzw. der $C_{4+}$-Kohlenwasserstoffe, kondensiert ist. Der partiell kondensierte Gasstrom wird in einem Abscheider 3 einer Phasentrennung unterzogen, wobei das Kondensat über Leitung 4 einer Rektifiziersäule 5 zugeleitet wird, in der es in eine $C_{2+}$-, $C_{3+}$- bzw. $C_{4+}$- Fraktion, die als Produktstrom über Leitung 6 aus dem Sumpf der Säule abgezogen wird, und in einen leichter siedende Anteile enthaltenden Restgasstrom 7 zerlegt wird. Die Rektifikation wird durchgeführt unter Verwendung einer mit Fremdkälte betriebenen Kopfkühlung 8 sowie einer beispielsweise mit Niederdruckdampf oder Heisswasser beheizten Sumpfheizung 9, in der ein von Leitung 6 abgezweigter Teilstrom des Sumpfprodukts aufgeheizt und dann wieder in den Säulensumpf zurückgeführt wird.

Die im Abscheider 3 nach Abtrennung des Kondensats verbliebene gasförmige Fraktion wird, bevor sie über Leitung 10 einer Expansionsturbine 11 zugeleitet wird, im oberen Bereich des Abscheiders 3 durch indirekten Wärmetausch mit der arbeitsleistend entspannten gasförmigen Fraktion im Wärmetauscher 20 abgekühlt. Die dabei auskondensierenden schweren Bestandteile fallen im Gegenstrom zur aufsteigenden gasförmigen Fraktion in den unteren Bereich des Abscheiders 3, wobei sich durch die Gegenstromführung von gebildetem Kondensat und gasförmiger Fraktion eine rektifikatorische Wirkung ergibt, die dem Einbau einiger Stoffaustauschböden entspricht.

Die weiter abgekühlte und kondensatfreie gasförmige Fraktion wird schliesslich über Leitung 10 zur Expansionsturbine 11 geleitet und nach arbeitsleistender Entspannung über Leitung 12 bei der tiefsten Verfahrenstemperatur abgezogen. Nach Erwärmung im Wärmetauscher 20 wird dieses Gas mit dem Restgas der Rektifikation aus Leitung 7 vermischt und im Wärmetauscher 2 auf annähernd Umgebungstemperatur angewärmt, bevor es über Leitung 13 als Restgas abgezogen wird.

Die über die Leitungen 4 und 10 aus dem Abscheider 3 bzw. über Leitung 7 aus der Rektifiziersäule 5 abgezogenen Ströme können vor ihrer weiteren Verarbeitung gegebenenfalls auf geeignete Temperaturniveaus angewärmt bzw. abgekühlt werden, was beispielsweise im Wärmetauscher 2 erfolgen kann. Für die Ströme in den Leitungen 4 und 7 ist dies durch gestrichelte Linien 14 bzw. 15 angedeutet. Der Fremdkältebedarf für das Verfahren wird durch einen Kältekreislauf gedeckt, der durch 16 schematisch angedeutet ist und die Kühlung des Gasgemisches im Wärmetauscher 2 gemeinsam mit anzuwärmenden Verfahrensströmen bewirkt.

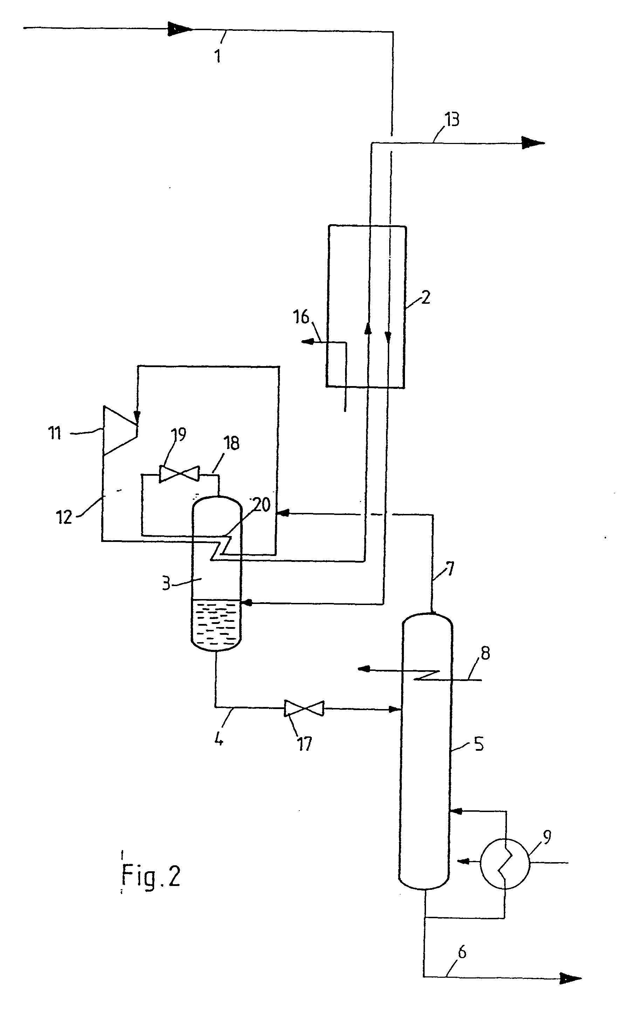

Bei dem in der Fig. 2 dargestellten Ausführungsbeispiel steht der zu zerlegende Gasstrom in Leitung 1 unter einem relativ hohen Druck. Nach der Abtrennung des Kondensats im Abscheider 3 wird dieses deshalb vor Einspeisung in die Rektifikation 5 auf den Rektifikationsdruck entspannt. Die gasförmige Fraktion aus dem Abscheider 3 wird über Leitung 18 abgezogen, im Ventil 19 auf den Druck des Restgases entspannt und nach Anwärmung im Wärmetauscher 20 gegen die noch nicht entspannte gasförmige Fraktion mit dem Restgas aus Leitung 7 vermischt, bevor das Gemisch der Turbine 11 zugeleitet wird. Durch die Anwärmung im Wärmetauscher 20 wird nicht nur die bei der Drosselentspannung 19 gewonnene Kälte an die gasförmige Fraktion im Abscheider 3 übertragen, sondern als wesentliches Moment auch eine Erwärmung der gasförmigen Fraktion über den Taupunkt hinaus bewirkt, so dass bei der Vermischung mit der Restgasfraktion 7 keine Kondensatbildung erfolgt.

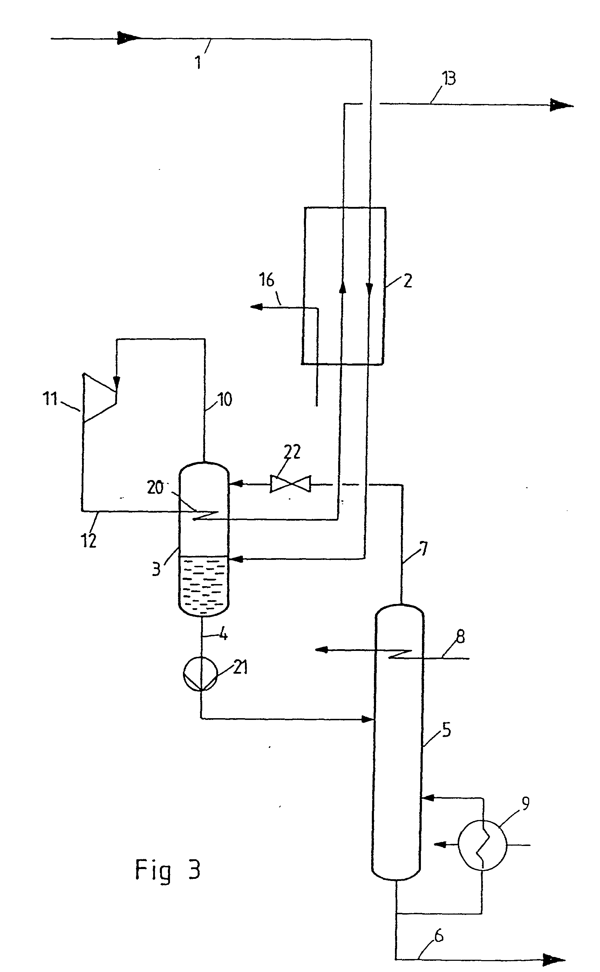

In einem konkreten Ausführungsbeispiel gemäss Fig. 3 wird ein Raffineriegas, das 20,1% Wasserstoff, 31,2% Methan, 13,3% Ethylen, 16,9% Ethan, 5,2% Propylen, 1,8% Propan, 0,9% $C_{4+}$-Kohlenwasserstoffe, 0,1% Schwefelverbindungen und 10,5% Inerte enthält, bei einem Druck von 16,2 bar und einer Temperatur von 288 K über Leitung 1 in die Anlage eingespeist. Das Gas, aus dem $C_{2+}$-Kohlenwasserstoffe zurückgewonnen werden sollen, wird im Wärmetauscher 2 auf 175 K abgekühlt und danach in den Abscheider 3 geleitet. Das über Leitung 4 abgezogene Kondensat wird in der Pumpe 21 auf den Rektifikationsdruck von 32 bar gepumpt und in die Kolonne 5, die bei einer Kopftemperatur von 180 K und einer Sumptemperatur von 285 K betrieben wird, eingespeist. Dieses Kondensat enthält 0,3% Wasserstoff, 20,1% Methan, 26,7% Ethylen, 35,0% Ethan, 10,9% Propylen, 3,8% Propan, 1,8% $C_{4+}$-Kohlenwasserstoffe, 0,2% Schwefelverbindungen und 1,2% Inerte. Über Leitung 6 wird ein Produktstrom mit nur 70 ppm Methan sowie 33,8% Ethylen, 45,0% Ethan, 14,0% Propylen, 4,8% Propan, 2,2% $C_{4+}$-Kohlenwasserstoffen und 0,2% Schwefelverbindungen abgezogen. Die $C_2$-Ausbeute beträgt 96,6% (bezogen auf den $C_2$-Gehalt im zu zerlegenden Gasstrom 1). Das Restgas der Rektifikation enthält 1,6% Wasserstoff, 91,0% Methan, 2,1% Ethylen, 0,3% Ethan und 5,0% Inerte. Bei einer Temperatur von 180 K wird es über Leitung 7 abgezogen, im Ventil 22 auf 16 bar entspannt und in den Abscheider 3 eingeführt. Das sich im oberen Bereich des Abscheiders 3 bildende Gasgemisch wird in der Turbine 11 auf einen Druck von 8,5 bar entspannt, wobei sich eine Temperatur von 140 K einstellt. Diese Spitzenkälte wird mittels des im Abscheider 3 befindlichen Wärmetauschers 20 an die gasförmige Fraktion übertragen, woraufhin das entspannte Gas bei einer Temperatur von 170 K zum Wärmetauscher 2 geleitet wird, bevor es schliesslich über Leitung 13 bei einer Temperatur von 285 K und einem Druck von 7,9 bar abgegeben wird.

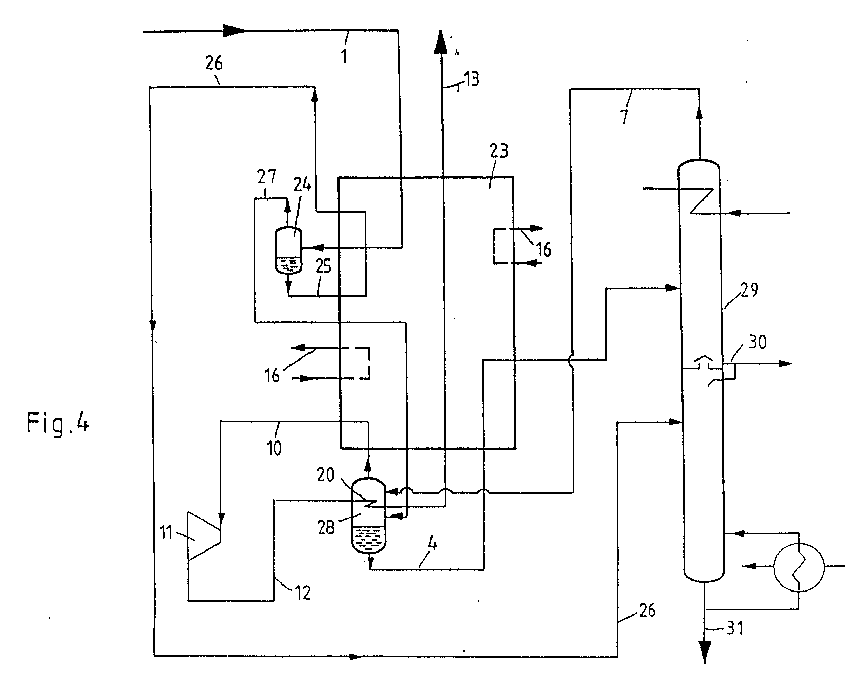

Das in Fig. 4 dargestellte Ausführungsbeispiel zeigt eine Variante des erfindungsgemässen Verfahrens, bei der in einem ersten Verfahrensschritt eine $C_{5+}$-Abtrennung aus dem Gasgemisch vorgenommen wird. Der Einsatzstrom 1 wird dazu in einem Wärmetauscher 23 zunächst nur so weit abgekühlt, dass der grösste Teil der $C_{5+}$-Kohlenwasserstoffe kondensiert. Das teilweise abgekühlte Gemisch wird bei einer Zwischentemperatur aus dem Wärmetauscher 23 herausgeführt, in einem Abscheider 24 einer Phasentrennung unterzogen, wobei die kondensierten Anteile über Leitung 25 abgetrennt, nach teilweiser Erwärmung im Wärmetauscher 23 über Leitung 26 abgezogen und einer Rektifikation zugeführt werden. Der gasförmig verbliebene Anteil des Gasstroms wird über Leitung 27 aus dem Abscheider 24 abgezogen, erneut im Wärmetauscher 23 abgekühlt und schliesslich dem Abscheider 28 zugeführt, der dem Abscheider 3 der vorher beschriebenen Ausführungsbeispiele entspricht.

Die Rektifikation der in den Abscheidern 24 und 28 abgetrennten Kondensate erfolgt in einer Trennsäule 29, die gegenüber der in den vorausgegangenen Beispielen eingesetzten Trennsäule eine grössere Anzahl von Böden aufweist. Zwischen den beiden Zuführungsleitungen 26 und 4 weist diese Säule eine Entnahmeleitung 30 an der Stelle auf, an der sich die höchste $C_3/C_4$-Konzentration befindet. Im Sumpf der Säule 29 fällt eine Flüssigkeit an, die im wesentlichen $C_{5+}$-Kohlenwasserstoffe enthält und die über Leitung 31 als Produktstrom abgezogen wird. Vom Kopf der Säule 29 wird über Leitung 7 wie in den vorausgegangenen Beispielen eine leichte Fraktion, die im wesentlichen $C_1$- und gegebenenfalls $C_2$-Kohlenwasserstoffe enthält, abgezogen und in den Abscheider 28 eingeleitet.

Bei diesem Verfahren werden die schweren Anteile, die im Abscheider 24 abgetrennt worden sind, ebenfalls der Rektifikation zugeführt. Auf diese Weise lässt sich mit relativ geringem Aufwand eine sehr hohe Ausbeute an $C_3$- und $C_4$-Kohlenwasserstoffen erzielen.

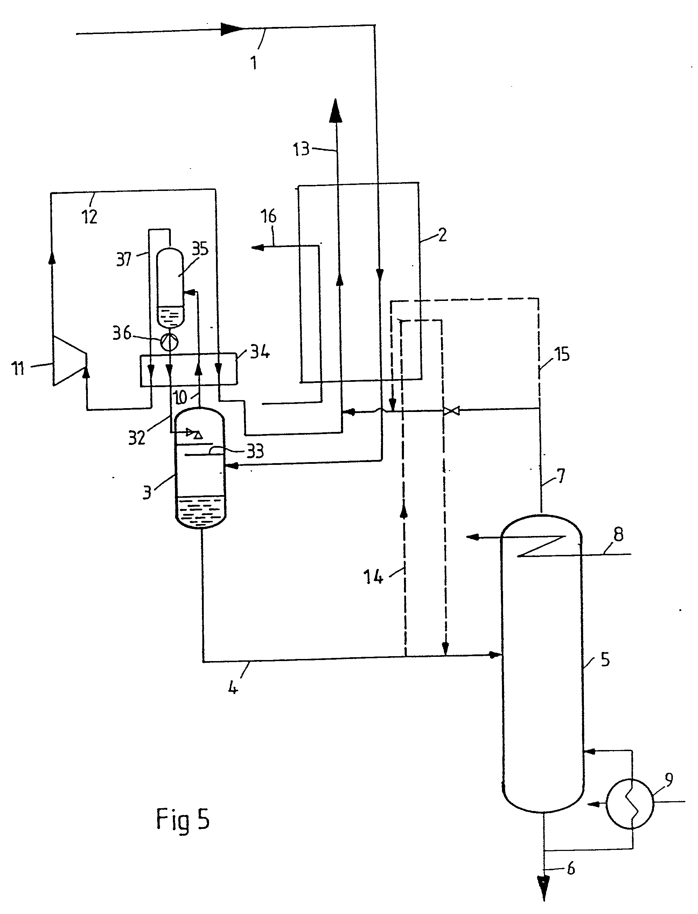

In der Fig. 5 ist eine Ausführungsform der Erfindung dargestellt, bei der die aus der gasförmigen Fraktion abgetrennten zusätzlich auskondensierten Komponenten in einem separaten Abscheider aufgefangen werden. Um den wesentlichen Unterschied in der Verfahrensführung aufzuzeigen, sei hier lediglich auf die Änderung gegenüber der in Fig. 1 dargestellten Verfahrensvariante eingegangen.

Nach der partiellen Kondensation im Wärmetauscher 2 wird im Abscheider 3 wiederum eine Phasentrennung vorgenommen. Im oberen Bereich des Abscheiders 3 ist jedoch nicht mehr ein gewickelter Wärmetauscher 20 vorgesehen. Statt dessen tritt die gasförmige Fraktion aus dem Abscheider 3 im oberen Bereich des Abscheiders in einen Stoffaustausch mit im Gegenstrom herabfliessendem, über Leitung 32 herangeführtem zusätzlichen Kondensat. Zur Unterstützung des Stoffaustausches können gegebenenfalls einige Böden 33, eine Schüttung oder dergleichen in diesem Bereich vorgesehen sein. Das über Leitung 10 aus dem oberen Bereich des Abscheiders 3 abgezogene Gas wird im Wärmetauscher 34, der beispielsweise als einfacher Plattenwärmetauscher ausgeführt sein kann, abgekühlt, wobei weitere Komponenten kondensieren und im Abscheider 35 abgetrennt werden. Das Kondensat wird über eine Pumpe 36, die die Druckverluste innerhalb der Leitungsführung überwindet, wieder durch den Wärmetauscher 34 geführt und dabei auf die Temperatur des Abscheiders 3 erwärmt, bevor es über Leitung 32 in den oberen Bereich des Abscheiders 3 eingespeist wird. Der im Wärmetauscher 34 nicht kondensierte Anteil der gasförmigen Fraktion gelangt über Leitung 37 zur Expansionsturbine 11 und wird dort arbeitsleistend entspannt. Das entspannte Gas wird über Leitung 12 abgezogen, gibt seine Spitzenkälte zunächst im Wärmetauscher 34 an die gasförmige Fraktion 10 ab und wird dann im Wärmetauscher 2 gegen partiell zu kondensierendes Gas angewärmt, bevor es schliesslich über Leitung 13 abgezogen wird.

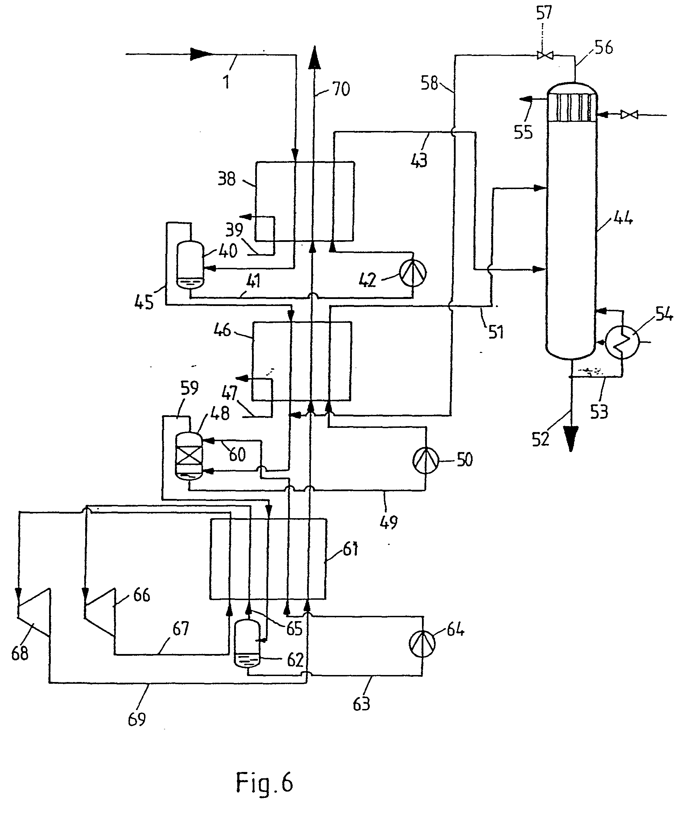

Das in der Fig. 6 dargestellte Ausführungsbeispiel eignet sich insbesondere zur $C_{2+}$-Abtrennung. Über Leitung 1 wird der zu zerlegende Gasstrom bei annähernd Umgebungstemperatur herangeführt und zunächst einer zweistufigen partiellen Kondensation unterzogen, wobei ein Grossteil der $C_{2+}$-Kohlenwasserstoffe kondensiert. Zunächst wird der Gasstrom im Wärmetauscher 38 gegen Fremdkälte 39 und gegen anzuwärmende Verfahrensströme abgekühlt und danach in einem Abscheider 40 einer ersten Phasentrennung unterzogen. Das abgetrennte Kondensat, das im wesentlichen aus $C_{2+}$-Kohlenwasserstoffen besteht und nur geringe Mengen leichtersiedender Komponenten enthält, wird über Leitung 41 abgezogen, mittels einer Pumpe 42 durch den Wärmetauscher 38 gefördert, indem es gegen abzukühlendes Gas wieder erwärmt wird und gelangt anschliessend über Leitung 43 in den unteren Bereich einer Rektifiziersäule 44. Die gasförmig verbliebene Fraktion aus dem Abscheider 40 wird über Leitung 45 abgezogen, in einem weiteren Wärmetauscher 46 gegen Fremdkälte 47 und anzuwärmende Verfahrensströme weiter abgekühlt und tritt danach in den unteren Bereich eines weiteren Abscheiders 48 ein. Kondensat aus dem Abscheider 48 wird über Leitung 49 abgezogen und mittels der Pumpe 50 durch den Wärmetauscher 46, in dem eine Anwärmung gegen abzukühlende Verfahrensströme erfolgt, gefördert und gelangt schliesslich über Leitung 51 ebenfalls in die Rektifiziersäule 44. Da dieser Strom einen grösseren Anteil leichter Komponenten enthält als der über Leitung 43 herangeführte Strom, erfolgt die Einspeisung in die Rektifiziersäule 44 an einer entsprechend dem Gleichgewichtsverlauf zu bestimmenden höheren Stelle. In der Rektifiziersäule wird eine $C_{2+}$Fraktion von leichtersiedenden Komponenten abgetrennt und über Leitung 52 aus dem Sumpf abgezogen. Ein Teil des Sumpfprodukts wird über Leitung 53 abgezweigt, im Wärmetauscher 54 erwärmt und als Sumpfheizung in den unteren Bereich der Säule zurückgeführt. Am durch Fremdkälte 55 gekühlten Kopf der Rektifiziersäule 44 fällt eine im wesentlichen nur noch Methan und leichtersiedende Komponenten enthaltende Fraktion an, die über Leitung 56 abgezogen wird. Nach Entspannung auf den Rohgasdruck im Ventil 57 wird das Kopfprodukt über Leitung 58 ins Rohgas zurückgeführt und durchläuft gemeinsam mit der aus dem Abscheider 40 abgezogenen gasförmigen Fraktion, nach dem diese im Wärmetauscher 46 weiter abgekühlt wurde, den Abscheider 48. Die sich im Abscheider 48 ausbildende gasförmige Fraktion, die also sowohl den im Wärmetauscher 46 nicht kondensierten Anteil der gasförmigen Fraktion aus dem Abscheider 40 als auch das Kopfprodukt 56 der Rektifikation 44 enthält, wird am oberen Ende des Abscheiders 48 über Leitung 59 abgezogen. Innerhalb des Abscheiders 48 tritt sie dabei zunächst in einen Gegenstrom-Stoffaustausch mit einer über Leitung 60 herangeführten, noch zu beschreibenden Kondensatfraktion, die sich im wesentlichen bei Siedetemperatur befindet. Die über Leitung 59 abgezogene gasförmige Fraktion wird dann in einem Wärmetauscher 61, der als Plattenwärmetauscher ausgebildet ist, gegen die bei einer nachfolgenden arbeitsleistenden Entspannung gebildete Spitzenkälte abgekühlt und teilweise kondensiert. Das dabei gebildete zusätzliche Kondensat wird in einem Abscheider 62 abgetrennt, über Leitung 63 abgezogen und mittels der Pumpe 64 durch den Wärmetauscher 61, indem es wieder bis auf etwa Siedetemperatur erwärmt wird, gefördert und schliesslich über Leitung 60 in den oberen Bereich des Abscheiders 48 eingespeist. Die gasförmige Fraktion aus dem Abscheider 62 wird über Leitung 63 zum Wärmetauscher 61 geführt und nach Ewärmung zweistufig arbeitsleistend entspannt. Dabei wird zunächst eine erste arbeitsleistende Entspannung 64 vorgenommen und das teilentspannte Gas über Leitung 65 erneut im Wärmetauscher 61 angewärmt, bevor es in der Expansionsmaschine 66 weiter entspannt wird. Das entspannte kalte Gas wird schliesslich über Leitung 67 abgezogen und in den Wärmetauschern 61, 46 und 38 gegen abzukühlende Verfahrensströme angewärmt und tritt dann als Leichtgasfraktion über Leitung 68 aus der Anlage aus.

In einem konkreten Ausführungsbeispiel gemäss Fig. 6 wird über Leitung 1 ein Gas bei einer Temperatur von 313 K und unter einem Druck von 21 bar herangeführt, das 22,5% Wasserstoff, 30,9% Methan, 23,7% $C_2$-Kohlenwasserstoffe, 6,8% $C_3$-Kohlenwasserstoffe, 12,5% $C_{4+}$-Kohlenwasserstoffe und 3,6% an restlichen Komponenten (im wesentlichen Stickstoff und Kohlenmonoxid, daneben geringe Mengen Kohlendioxid und Sauerstoff) enthält. Nach Abkühlung auf 237 K wird im Abscheider 40 ein erstes Kondensat abgetrennt, das neben $C_{2+}$-Kohlenwasserstoffen nur noch 7,6% Methan und 0,5% Wasserstoff sowie 0,4% restliche Komponenten enthält. Die verbleibende Gasphase wird im Wärmetauscher 46 auf eine Temperatur von 176 K abgekühlt und nach Vermischung mit dem Kopfprodukt der Rektifikation, das 2,6% Wasserstoff, 92,5% Methan, 2,4% $C_2$-Kohlenwasserstoffe und 2,5% restliche Komponenten enthält, in den Abscheider 48 eingeführt. Die über Leitung 59 aus dem Abscheider 48 abgezogene gasförmige Fraktion enthält 35,1% Wasserstoff, 55,9% Methan, 3,4% $C_2$-Kohlenwasserstoffe und 5,6% restliche Komponenten. Es fällt bei einer Temperatur von 169 K an und wird im Wärmetauscher 61 auf 155 K abgekühlt. Dabei bildet sich ein Kondensat, das 1,0% Wasserstoff, 73,8% Methan, 23,1% $C_2$-Kohlenwasserstoffe und 2,1% restliche Komponenten enthält und das nach erneuter Erwärmung im Wärmetauscher 61 auf den Kopf des Abscheiders 48 aufgegeben wird. Bei Einspeisung in den Abscheider 48 ist das Kondensat bis auf Siedetemperatur angewärmt, jedoch noch überwiegend im flüssigen Zustand. Die im Abscheider 62 anfallende gasförmige Fraktion, die anschliessend arbeitsleistend entspannt und schliesslich als Restgasfraktion 68 abgegeben wird, enthält 39,5% Wasserstoff, 53,6% Methan, 0,9% $C_{2+}$-Kohlenwasserstoffe und 6,0 restliche Komponenten. Bei der Rektifikation 44 fällt neben der schon erwähnten Kopffraktion ein Sumpfprodukt über Leitung 52 an, das 0,8% Methan, 54,0% $C_2$-Kohlenwasserstoffe, 15,8% $C_3$-Kohlenwasserstoffe, 29,0% $C_{4+}$-Kohlenwasserstoffe und 0,4 % restliche Komponenten enthält. Es wird bei einer Sumpftemperatur von 304 K aus der Rektifiziersäule, die bei einem Druck von 30 bar betrieben wird, abgezogen. Dieser Strom enthält 98,8% der über Leitung 1 in die Anlage eingeführten $C_{2+}$-Kohlenwasserstoffe.

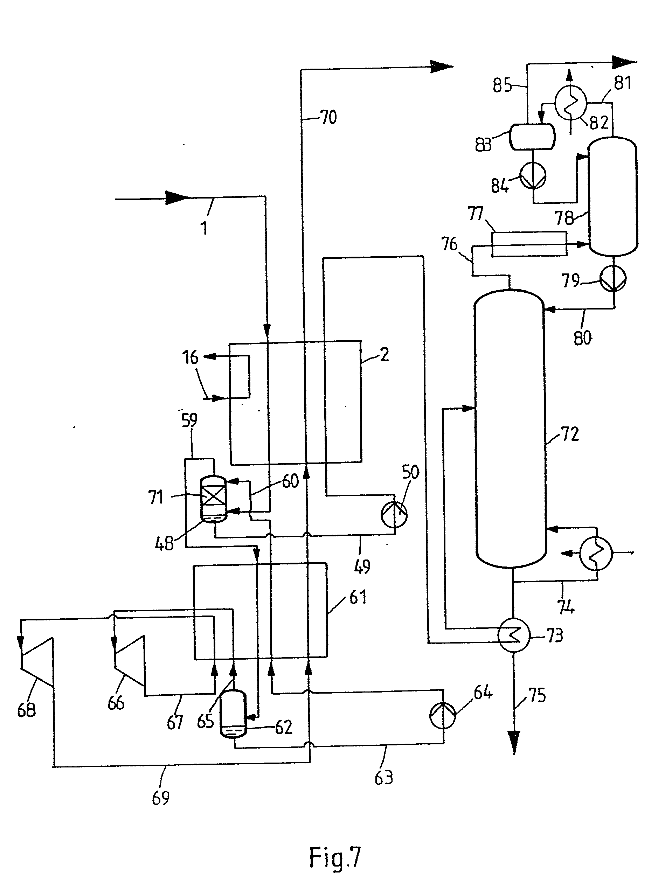

Bei der in Fig. 7 dargestellten Ausführungsweise der Erfindung wird ebenso wie beim Verfahren gemäss Fig. 6 eine separate Abtrennung der zusätzlich kondensierten Komponenten vorgenommen. Die in der Fig. 7 dargestellte Ausführungsform eignet sich insbesondere für die Abtrennung von $C_{4+}$-Kohlenwasserstoffen. Die Verfahrensführung entspricht in wesentlichen Teilen denen der Fig. 6, so dass hier nur die Unterschiede dazu aufgezeigt werden.

Der über Leitung 1 zugeführte Rohgasstrom wird einstufig partiell kondensiert und tritt somit nach Durchströmen des Wärmetauschers 2 ohne Zumischung einer weiteren Fraktion in den Abscheider 48 ein. Die nach Durchströmen des Rektifikationsabschnitts 71 im Abscheider 48 anfallende gasförmige Fraktion wird über Leitung 59 abgezogen und entsprechend der Ausführungsform von Fig. 6 weiterverarbeitet, wobei der einzige Unterschied darin besteht, dass die über Leitung 65 aus dem Abscheider 62 abgezogene gasförmige Fraktion vor ihrer arbeitsleistenden Entspannung jeweils nur einen Teil des Wärmetauschers 61 durchströmt, also nicht bis auf die Eintrittstemperatur des Wärmetauschers 61 angewärmt wird. Die Kondensatfraktion aus dem Abscheider 48 wird über Leitung 49 abgezogen, auf den Druck der Rektifikation 72 gepumpt und nach Anwärmung im Wärmetauscher 2 vor Einspeisung in die Rektifiziersäule noch im Wärmetauscher 73 gegen Sumpfprodukt der Rektifikation weiter angewärmt. Während ein Teilstrom 74 des Sumpfprodukts zur Sumpfbeheizung erwärmt und wieder in den Kolonnensumpf zurückgeführt wird, tritt der Rest des Sumpfprodukts über Leitung 75 als $C_{4+}$-Produktfraktion aus der Anlage aus. Am Kopf der Rektifiziersäule 72 wird über Leitung 56 eine $C_{3-}$-Fraktion abgezogen, die in einem Wärmetauscher 77 gekühlt und partiell kondensiert wird. Als Kühlmittel kann dabei beispielsweise Umgebungsluft verwendet werden. Der kondensierte Anteil wird in einem Abscheider 78 abgetrennt und über eine Pumpe 79 und über Leitung 80 als Rücklaufflüssigkeit auf den Kopf der Rektifiziersäule 72 gefördert. Der nichtkondensierte Anteil tritt über Leitung 81 in einen Kühler 82, wobei weitere Bestandteile kondensieren. Das dabei gebildete Kondensat wird im weiteren Abscheider 83 abgetrennt und mittels der Pumpe 84 in den oberen Bereich des Abscheiders 78 zurückgeführt. Über Leitung 85 wird eine $C_{3-}$-Kohlenwasserstoff-Fraktion als weitere Produktfraktion aus dem Abscheider 83 abgezogen.

In einem konkreten Ausführungsbeispiel gemäss Fig. 7 wird über Leitung 1 ein Rohgas bei einer Temperatur von 313 K und unter einem Druck von 12 bar in den Wärmetauscher 2 geführt und dort auf 234 K abgekühlt. Die aus dem Abscheider 48 über Leitung 59 abgezogene gasförmige Fraktion enthält 80,8% Wasserstoff, 15,5% Methan, 0,7% $C_2$-Kohlenwasserstoffe, 0,5% $C_3$-Kohlenwasserstoffe und 2,5% $C_4$-Kohlenwasserstoffe. Nach Abkühlung auf 190 K im Wärmetauscher 61 fällt in Leitung 63 ein Kondensat an, das 0,4% Wasserstoff, 3,2% Methan, 4,0% $C_2$-Kohlenwasserstoffe, 12,7% $C_3$-Kohlenwasserstoffe und 79,5% $C_4$-Kohlenwasserstoffe enthält. Es wird nach Erwärmung auf 230 K über Leitung 60 in den oberen Bereich des Abscheiders 48 eingespeist. Dabei befindet es sich im wesentlichen an seiner Siedetemperatur, liegt aber noch in flüssigem Zustand vor. Die aus dem Abscheider 62 abgezogene gasförmige Fraktion enthält 83,3% Wasserstoff, 15,9% Methan, 0,6% $C_2$-Kohlenwasserstoffe und je 0,1% $C_3$-Kohlenwasserstoffe und $C_4$-Kohlenwasserstoffe. Die arbeitsleistend entspannte gasförmige Fraktion enthält lediglich etwa 0,2% der über Leitung 1 zugeführten $C_4$-Kohlenwasserstoffe. Das aus dem Abscheider 48 abgezogene Kondensat enthält 0,5% Wasserstoff, 1,6% Methan, 1,0% $C_2$-Kohlenwasserstoffe, 4,4% $C_3$-Kohlenwasserstoffe und 92,5% $C_4$-Kohlenwasserstoffe und wird anschliessend in der Rektifiziersäule 72 weiter zerlegt.

## Patentansprüche

1. Verfahren zur Abtrennung von $C_{2+}$-, $C_{3+}$- oder von $C_{4+}$-Kohlenwasserstoffen aus einem leichte Kohlenwasserstoffe und gegebenenfalls leichter als Methan siedende Komponenten enthaltenden Gasstrom, bei dem der unter erhöhtem Druck stehende Gasstrom (1) abgekühlt, partiell kondensiert und in einem Abscheider (3, 28, 48) in eine flüssige und eine gasförmige Fraktion getrennt wird, bei dem die gasförmige Fraktion (10, 18, 65) arbeitsleistend entspannt und die flüssige Fraktion (4, 49) durch Rektifikation (5, 29, 44, 72) in einem im wesentlichen $C_{2+}$-, $C_{3+}$- bzw.

$C_{4+}$-Kohlenwasserstoffe enthaltenden Produktstrom (6, 30, 52, 75) und einen überwiegend leichter siedende Komponenten enthaltenden Restgasstrom (7, 56, 85) zerlegt wird und bei dem die nach der partiellen Kondensation anfallende gasförmige Fraktion vor der arbeitsleistenden Entspannung durch Wärmetausch (20, 34, 61) mit der arbeitsleistend entspannten gasförmigen Fraktion (12, 67, 69) weiter abgekühlt wird und die dabei zusätzlich auskondensierenden Komponenten vor der arbeitsleistenden Entspannung (11, 66) abgetrennt werden, dadurch gekennzeichnet, dass der Abscheider (3, 28, 48), in dem die Trennung in die flüssige und die gasförmige Fraktion durchgeführt wird, mindestens zwei Gleichgewichtsstufen aufweist und dass die zusätzlich auskondensierten Komponenten im Gegenstrom zu der in dem Abscheider (3, 28, 48) aufsteigenden gasförmigen Fraktion in den unteren Bereich des Abscheiders fallen.

2. Verfahren nach Anspruch 1, dadurch gekennzeichnet, dass der Wärmetausch (20) zwischen der nicht entspannten und der entspannten gasförmigen Fraktion im oberen Bereich des Abscheiders (3, 28, 48) durchgeführt wird.

3. Verfahren nach Anspruch 1, dadurch gekennzeichnet, dass der Wärmetausch (34, 61) zwischen der nicht entspannten und der entspannten gasförmigen Fraktion ausserhalb des Abscheiders (3, 48) durchgeführt wird und die dabei zusätzlich auskondensierten Komponenten (32, 63) in den Abscheider (3, 48) zurückgeführt werden.

4. Verfahren nach Anspruch 3, dadurch gekennzeichnet, dass die zusätzlich auskondensierten Komponenten (32) vor der Rückführung in den Abscheider (28, 48) wieder teilweise erwärmt werden.

5. Verfahren nach Anspruch 4, dadurch gekennzeichnet, dass die Erwärmung mindestens bis zum Siedepunkt der zusätzlich auskondensierten Komponenten erfolgt.

6. Verfahren nach einem der Ansprüche 1 bis 5, dadurch gekennzeichnet, dass die flüssige Fraktion (4, 49) vor der Rektifikation (29, 44, 72) mindestens teilweise gegen den abzukühlenden Gasstrom (1) erwärmt wird.

7. Verfahren nach einem der Ansprüche 1 bis 6, dadurch gekennzeichnet, dass bei der Verarbeitung eines Gasstroms, der reich an leichter als Methan siedenden Komponenten ist, vor der arbeitsleistenden Entspannung (11, 66) der gasförmigen Fraktion (10, 59) aus dieser $C_1$- und $C_2$-Kohlenwasserstoffe durch partielle Kondensation abgetrennt werden (35, 62).

8. Verfahren nach einem der Ansprüche 1 bis 7, dadurch gekennzeichnet, dass vor der Bildung der flüssigen und der gasförmigen Fraktion ein Grossteil von gegebenenfalls im Gasstrom enthaltenen $C_{5+}$-Kohlenwasserstoffen abgetrennt wird (24).

9. Verfahren nach Anspruch 8, dadurch gekennzeichnet, dass die abgetrennten $C_{5+}$-Kohlenwasserstoffe (25) ebenfalls der Rektifikation zugeführt werden, dass die Einspeisung in eine Rektifiziersäule (29) unterhalb der Einspeisung der bei der partiellen Kondensation gebildeten flüssigen Fraktion (4) erfolgt und dass ein im wesentlichen $C_3$- und $C_4$-Kohlenwasserstoffe enthaltender Strom (30) zwischen den beiden Einspeisungen entnommen wird.

## Claims

1. A process for separating $C_{2+}$-, $C_{3+}$- or $C_{4+}$-hydrocarbons from a gas stream containing light hydrocarbons and possibly components which are lower-boiling than methane, wherein the gas stream (1) which is under increased pressure is cooled, partially condensed and separated in a separator (3, 28, 48) into a liquid fraction and a gaseous fraction, wherein the gaseous fraction (10, 18, 65) is expanded with the production of work and the liquid fraction (4, 49) is separated by rectification (5, 29, 44, 72) into a product stream (6, 30, 52, 75) containing fundamentally $C_{2+}$-, $C_{3+}$- or $C_{4+}$-hydrocarbons and a residual gas stream (7, 56, 85) containing predominantly lower-boiling components, and wherein the gaseous fraction which is produced after the partial condensation is further cooled, prior to the work-producing expansion, by heat exchange (20, 34, 61) with the gaseous fraction (12, 67, 69) which is expanded with the production of work, and the components which have additionally condensed out are separated prior to the work-producing expansion (11, 66), characterised in that the separator (3, 28, 48) in which the separation into the liquid and gaseous fractions is carried out, comprises at least two equilibrium stages and that the components which have additionally condensed out fall into the lower region of the separator in counter-flow to the gaseous fraction ascending in the separator (3, 28, 48).

2. A process as claimed in claim 1, characterised in that the heat exchange (20) between the non-expanded gaseous fraction and the expanded gaseous fraction takes place in the upper region of the separator (3, 28, 48).

3. A process as claimed in claim 1, characterised in that the heat exchange (34, 61) between the non-expanded gaseous fraction and the expanded gaseous fraction takes place outside the separator (3, 48) and that the components (32, 63) which have thereby additionally condensed out are returned to the separator (3, 48).

4. A process as claimed in claim 3, characterised in that before being returned to the separator (28, 48) the components (32) which have additionally condensed out are partially reheated.

5. A process as claimed in claim 4, characterised in that the heating takes place at least to the boiling point of the components which have additionally condensed out.

6. A process as claimed in one of the claims 1 to 5, characterised in that prior to the rectification (29, 44, 72) the liquid fraction (4, 49) is heated at least partially in the presence of the gas stream (1) which is to be cooled.

7. A process as claimed in one of the claims 1 to 6, characterised in that in the case of the processing of a gas stream which is rich in components which are lower-boiling than methane, prior to the work-producing expansion (11, 66) of the gaseous fraction (10, 59), $C_1$- and $C_2$-hydrocarbons are separated therefrom by means of partial condensation (35, 62).

8. A process as claimed in one of the claims 1 to 7, characterised in that prior to the formation of the liquid fraction and the gaseous fraction a majority of $C_{5+}$-hydrocarbons possibly contained in the gas stream are separated (24).

9. A process as claimed in claim 8, characterised in that the separated $C_{5+}$-hydrocarbons (25) are likewise conducted to the rectification stage, that the input into a rectifier column (29) takes place below the input of the liquid fraction (4) formed during the partial condensation, and that a stream (30) fundamentally containing $C_3$- and $C_4$-hydrocarbons is withdrawn between the two inputs.

**Revendications**

1. Procédé de séparation d'hydrocarbures en $C_{2+}$, $C_{3+}$ ou $C_{4+}$ d'un courant contenant un hydrocarbure léger et, le cas échéant des constituants à point d'ébullition inférieur à celui du méthane, dans lequel ce courant gazeux (1), se trouvant sous haute pression, est refroidi, partiellement condensé et divisé dans un séparateur (3, 28, 48) en une fraction liquide et une fraction gazeuse, dans lequel ladite fraction gazeuse (10, 18, 65) subit une détente avec production de travail et ladite fraction liquide (4, 49) est séparée par rectification (5, 29, 44, 72) en un courant de produits (6, 20, 52, 75) essentiellement formé d'hydrocarbures en $C_{2+}$, $C_{3+}$ ou $C_{4+}$ et en un courant gazeux résiduel 67, 56, 85) contenant des constituants à point d'ébullition nettement inférieur, et dans lequel la fraction gazeuse restant après condensation partielle est soumise, préalablement à la détente avec production de travail, à un refroidissement supplémentaire, par échange de chaleur (20, 34, 61) avec la fraction gazeuse (12, 67, 69) détendue et ayant produit ledit travail et dans lequel les constituants qui ont été ainsi ultérieurement condensés sont séparés avant que ne se produise ladite détente avec production de travail (11, 66), ce procédé étant caractérisé en ce que le séparateur (3, 28, 48) dans lequel ladite séparation en phase liquide et en phase gazeuse est effectuée comporte au moins deux étages au niveau desquels règnent deux états d'équilibre thermodynamique différents et en ce que les constituants ainsi ultérieurement condensés tombent, à contrecourant de la fraction gazeuse ascendante, dans le séparateur, au niveau de la partie inférieure de ce dernier.

2. Procédé selon la revendication 1, caractérisé en ce que l'échange de chaleur (20) entre la fraction gazeuse non détendue et la fraction gazeuse détendue se produit dans la zone supérieure du séparateur (3, 28, 48).

3. Procédé selon la revendication 1, caractérisé en ce que l'échange de chaleur (34, 61) entre la fraction gazeuse non détendue et la fraction gazeuse détendue se produit à l'extérieur du séparateur (3, 48) et en ce que les constituants ultérieurement condensés (32, 62) sont renvoyés vers le séparateur (3, 48).

4. Procédé selon la revendication 3, caractérisé en ce que les constituants ultérieurement condensés (32) sont préalablement à leur retour dans le séparateur (28, 48), partiellement réchauffés.

5. Procédé selon la revendication 4, caractérisé en ce que le réchauffement est effectué au moins jusqu'au point d'ébullition des constituants ultérieurement condensés.

6. Procédé selon l'une des revendications 1 à 5, caractérisé en ce que la fraction liquide (4, 49) préalablement à la rectification (29, 44, 72) est au moins partiellement réchauffée à contrecourant du courant de gaz à refroidir (1).

7. Procédé selon l'une des revendications 1 à 6, caractérisé en ce qu'au cours du traitement d'un courant de gaz riche en constituants à température d'ébullition inférieure à celle du méthane, les hydrocarbures en $C_1$ et en $C_2$ sont séparés de la fraction gazeuse (10, 59) par condensation partielle avant détente, avec production de travail (11, 66), de cette fraction gazeuse.

8. Procédé selon l'une des revendications 1 à 7, caractérisé en ce que la plus grande partie des hydrocarbures en $C_{5+}$ contenus, le cas échéant, dans le courant de gaz sont séparés (24) avant formation des fractoins liquide et gazeuse.

9. Procédé selon la revendication 8, caractérisé en ce que l'on soumet également à la rectification les hydrocarbures en $C_{5+}$ séparés (25), en ce qu'ils alimentent une colonne de rectification (29) au-dessous du niveau d'alimentation de la fraction liquide (4) formée par condensation partielle et caractérisé en ce que l'on prélève entre les deux niveaux d'alimentation, un courant (30) contenant essentiellement des hydrocarbures en $C_3$ et en $C_4$.

Fig.1

Fig.2

Fig 3

Fig.4

EP 0 185 202 B1

Fig 5

Fig.6

21

Fig.7